Europäisches Patentamt

European Patent Office

Office européen des brevets

(11) Publication number: **0 361 548**
**A2**

(12) # EUROPEAN PATENT APPLICATION

(21) Application number: **89122017.0**

(22) Date of filing: **07.08.85**

(51) Int. Cl.5: **A61M 25/06**

(30) Priority: **07.08.84 JP 165452/84**

(43) Date of publication of application:
**04.04.90 Bulletin 90/14**

(60) Publication number of the earlier application in accordance with Art.76 EPC: **0 171 077**

(84) Designated Contracting States:
**BE DE FR GB IT SE**

(71) Applicant: **TERUMO KABUSHIKI KAISHA trading as TERUMO CORPORATION**
**44-1, 2-chome, Hatagaya Shibuya-Ku**
**Tokyo 151(JP)**

(72) Inventor: **Suzuki, Tatsuo**
**1-5-27-302, Sakuradai Oyamada**
**Machida-shi Tokyo(JP)**
Inventor: **Matsumoto, Atsushi Terumo**
**Kabushiki K.-Fujimi-Ryo**
**2517 Oomiya Fujinomiya-shi**
**Shizuoka-ken(JP)**

(74) Representative: **Henkel, Feiler, Hänzel & Partner**
**Möhlstrasse 37**
**D-8000 München 80(DE)**

(54) Catheter introducer device.

(57) The invention is directed to a catheter introducer device, comprising: a sheath portion (2) including a sheath (5) and a first hollow hub (4) fitted to a proximal end of said sheath (5) which is adapted to be inderted into blood vessels (18); a dilator (3) including a tube (7) detachably insertable into said sheath (5), and a second hub (6) fixed to a proximal end of said tube (7) which is adapted to guide a guide wire therethrough; first means for preventing relative axial movement of said sheath portion (2) and said dilator (3) when said tube (7) is inserted into said sheath (5) and second means for preventing relative circumferential rotation of said sheath portion (2) and said dilator (3) when said tube is inserted into said sheath (5), said first and second means being arranged betweeen said first and second hubs (4,5). In accordance with the invention said first and second means (9,10,11,12) are adapted to safely prevent relative axial movement and circumferential rotation of the sheath portion relative to the dilator.

F I G. 3

F I G. 4

## Catheter introducer device

The present invention relates to a catheter introducer device defined by the precharacterizing features of the claim.

A catheter introducer device is known as a device for percutaneous insertion of a catheter into a blood vessel. Such a device is composed of a sheath portion having a sheath and a sheath hub, and a dilator portion having a tube and a dilator hub. When such a catheter introducer device is used, the dilator portion is set in the sheath portion and the dilator and sheath portions are inserted into a blood vessel via a guide wire.

The dilator and sheath portions can be easily inserted when they are simultaneously rotated during insertion. In a conventional catheter introducer device, no means for simultaneously rotating the sheath and dilator portions is included. In such a conventional catheter introducer device, in order to insert the assembly of the sheath and dilator portions into a blood vessel while preventing rotation between the sheath and dilator portions, the hubs in the two portions must either be clamped together, or the tube of the dilator portion must be firmly clamped at a position above the sheath of the sheath portion so as to simultaneously rotate the two portions, thus resulting in a complex procedure.

Yet, even if such complex operation is performed, only the sheath portion can be inserted, the dilator portion being impeded by resistance of tissue of the like.

A catheter introducer device of the kind defined at the preamble is known from the EP-A-64 212. The second hub of this known device is screwed on the first hollow hub. The threads of this screw, which are arranged between the first and second hubs, form first and second means for preventing the relative axial movement and the relative circumferntial rotation, if the second hub is completey screwed on the first hub. If, however, the second hub is not completely screwed into the first hub, axial movement as well as circumferential rotation therebetween and hence between the sheath portion and the dilator is not prevented. Further, the second hub needs to be tightly screwed into the first hub in order to prevent loosening of the screw connection. A loos fit of the two hubs, as aforementioned, however, is not suited to prevent axial movement and circumferential rotation.

The catheter introducer device known from the DE-A-2 305 640 comprises a male-female tapering which is intended to hold the two parts of this catheter introducer device together. This tapering constitutes means for preventing relative axial movement and circumferential rotation when tightly assembled. This male-female tapering has a circular cross section and hence does not provide a safe connection for the two parts of the catheter introducer device, which may be loosened upon manipulation of the device.

It is the object of the present invention to provide a catheter introducer device of the kind defined at the preamble with safe means for preventing relative axial movement and circumferential rotation of the sheath portion relative to the dilator.

This object is attained by the characterizing features of the claim.

This invention can be more fully understood from the following detailed description when taken in conjunction with the accompanying drawings, in which:

Fig. 1 is a side view of an assembled catheter introducer device of the present invention,

Fig. 2 is a partial sectional view of the main body of the device shown in Fig.1,

Fig. 3 is a partial end view of the main body shown in Fig. 2, as viewed from the left side,

Fig 4 is a side view of the rod member in the device shown in Fig.1 and

Fig.5 to 7 are diagrams for explaining the operation for inserting the device shown in the preceeding figures into a blood vessel.

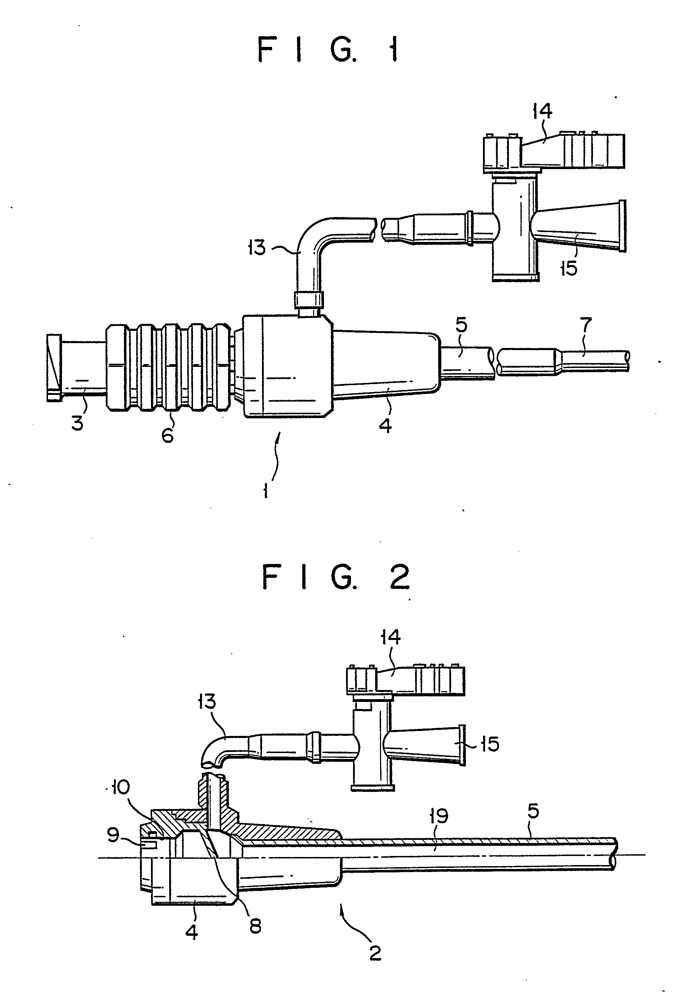

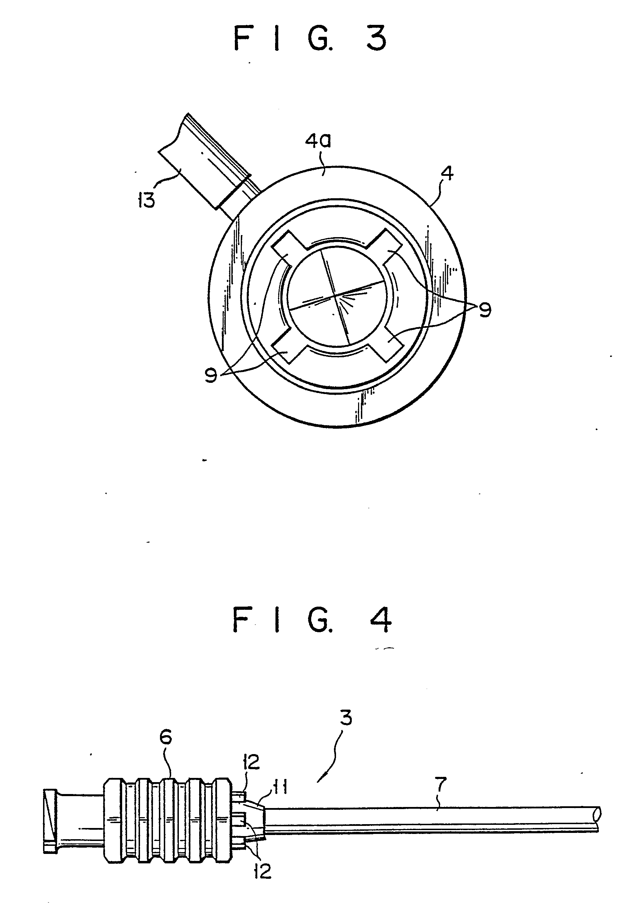

The catheter device 1 shown in Fig. 1 includes a main body 2 (Fig.2) and a rod member 3 (Fig. 4). The main body 2 corresponds to the sheath portion, and the rod member 3 corresponds to the dilator portion of a catheter introducer.

The main body 2 has a hub 4 and a sheath 5 having its proximal portion fixed and supported by the hub 4, as shown in Fig.2 . The hub 4 and the sheath 5 have a through path 19 for receiving a tube 7 of the rod member 3.

A valve 8 is mounted, preferably, in the portion of the through path 19 in the hub 4. The valve 8 serves to prevent reverse flow of blood when the medical device main body is inserted into a blood vessel.

As shown in Fig. 4, the rod member or dilator portion 3 has a hub 6 and a tube 7 fixed and supported thereby. The dilator portion 3 is inserted in and assembled with the main body 2 shown in Fig. 2. With this arrangement, when the main body 2 and the rod member 3 are percutaneously inserted into a blood vessel, they rotate relative to each other and, as such, may not lead themselves to easy insertion, as desribed above.

In view of this problem, according to the present invention a mechanism for preventing relative rotation between the main body 2 and the dilator portion 3 is incorporated.

Said mechanism is provided between the main body 2 and the dilator portion 3 and comprises a groove formed in one of the main body 2 and the dilator portion 3, and a rib, capable of being fitted within the groove, is formed on the other. The two members are taper fitted to prevent relative rotation and axial separation therebetween.

As shown in Fig. 2 and 3 in particular, at the rear end of the hub 4 of the main body 2, the inner wall of a hub 4 defining the rear end of the through path 19 constitutes a female taper fitting portion 10. At least one groove 9 (four grooves in Fig. 3) is formed at part of the fitting portion 10, in, preferably, a symmetrical configuration. When the grooves 9 are symmetrical, assembly is easy.

The main body 2 is connected at its hub portion 4 to a three port connection valve 14 through a side tube 13 communicating with the hub 4. This valve 14 can be operated to replenish a liquid through a lure taper port 15.

As shown in Fig. 4, at the side of the dilator portion, a male taper fitting portion 11 is formed at the distal end side of the dilator hub 6, and can be fitted within the fitting portion 10 of the sheath portion 2. Ribs 12 for fitting within the grooves 9 of the sheath portion 2 are formed on the fitting portion 11.

Fig. 1 shows a state wherein the sheath portion 2 in Fig. 2 is assembled with the dilator portion 3 shown in Fig. 4. Assembly can be performed as follows.

The tube 7 of the dilator portion 3 is inserted into the through path 19 in the hub 4 and the sheath 5 from the side of the hub 4 of the sheath portion 2. The hub 6 of the dilator portion 3 and the hub 4 of the sheath portion 3 are then fit together. At this time, the fitting portion 11 of the dilator portion 3 is fitted within the fitting portion 10 of the sheath portion 3 so as to prevent axial relative movement between the dilator and sheath portions 3 and 2. Similarly, the ribs 12 of the dilator portion 3 are tightly fitted into the grooves 9 of the sheath portion 2 and locked so as to prevent circumferential relative rotation between the dilator and sheath portions 3 and 2.

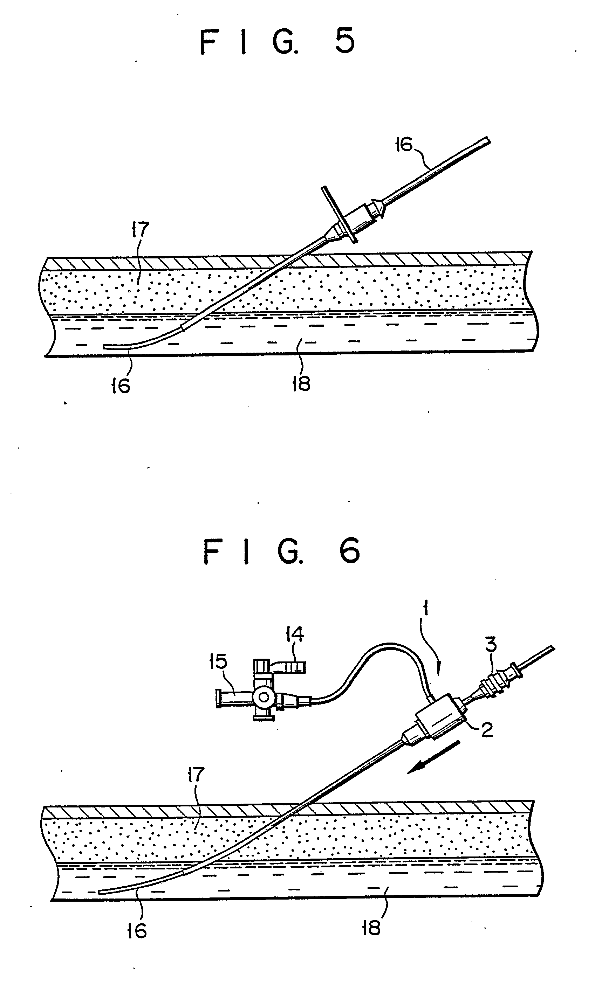

The method of using the device described above will be described with reference to Fig. 5 to 7.

A guide wire 16 is inserted into a blood vessel 18 through a subcutaneous tissue 17 by a suitable method such as the Seldinger method (Fig. 5). Then, the device main body 1, obtained by assembling the sheath portion (device main body) 2 shown in Fig. 2 and the dilator portion (rod member 3) shown in Fig. 4 so as to prevent axial movement and circumenferential rotation as shown in Fig. 1, is inserted into a blood vessel 18 by inserting the guide wire 16 into the tube 7, the sheath 5 and the through hole in the hubs 4 and 6 (Fig. 6).

In this process, insertion into the blood vessel can be easily performed when the hub 6 of the dilator portion 3 is rotated during insertion.

In the catheter introducer device of the present invention, relative rotation between the dilator and sheath portions 3 and 2 is prevented by the ribs 12 and grooves 9. In addition, axial movement between the portions 3 and 2 is prevented by a fitting force obtained by the female and male fitting portions 10 and 11. Therefore, insertion of the device main body 1 into the blood vessel 18 through means of the guide wire 16 is facilitated.

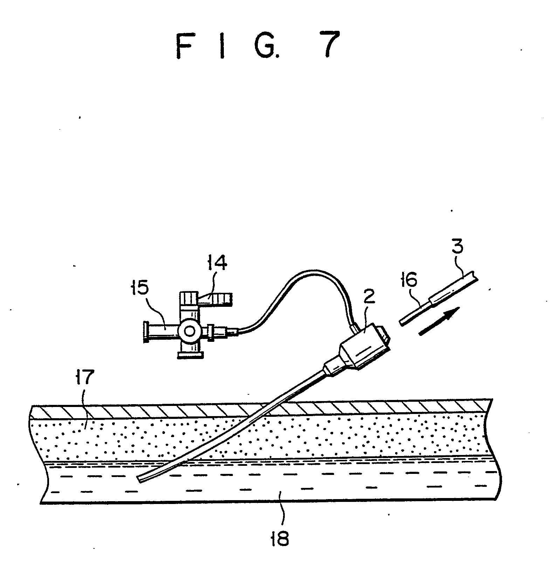

Subsequently, the sheath portion 6 is left in the blood vessel 18, and the guide wire 16 and the dilator portion 3 are pulled out (Fig. 7).

The catheter can be easily inserted into the blood vessel 18 through the indwelling sheath portion 2. Since the sheath portion 2 has a check valve 8, reverse flow of blood is prevented. If required, suction of a thrombus or injection of a physological saline solution can be performed through the lure taper port 15 of the sheath portion 2 before or after insertion of the catheter.

In the medical device of the present invention a female taper fitting portion and a groove are formed at the side of the main body, and a male fitting portion and a rib are formed at the side of the rod member. When the main body and the rod member are assembled, the taper fitting portions of the two members are tightly fit together so as to prevent relative axial movement or separation of the two members. At the same time, the groove and the rib of the two members are tightly fit together so as to prevent relative rotation between the two members. A similiar effect can be obtained if the means for preventing axial movement is a recess and a projection formed, respectively, in the fitting surfaces of the outer and inner cylinder hubs, while the means for preventing circumferential relative rotation is a female polygonal cylinder defining the hollow portion of the outer cylinder hub, and a male polygonal prism of the inner cylinder hub to be fitted therewith.

## Claims

A catheter introducer device, comprising:
a sheath portion (2) including a sheath (5) and a first hollow hub (4) fitted to a proximal end of said sheath (5) which is adapted to be inderted into blood vessels (18);
a dilator (3) including a tube (7) detachably insertable into said sheath (5), and a second hub (6) fixed to a proximal end of said tube (7) which is adapted to guide a guide wire therethrough;
first means for preventing relative axial movement of said sheath portion (2) and said dilator (3) when said tube (7) is inserted into said sheath (5) and second means for preventing relative circumferential rotation of said sheath portion (2) and said dilator (3) when said tube is inserted into said sheath (5),

said first and second means being arranged betweeen said first and second hubs (4,5),

characterized

in that said second hub (6) includes a male tapered section (11) and at least one rib (12) thereon,

in that said first hub (4) includes a female tapered inner surface (10) and at least one groove (9) formed therein, and

in that said male tapered section (11) is formed to fit into said female tapered inner surface (10) and said rib (12) is formed to fit into said groove (9) so that said relative axial movement and said relative rotation of said sheath portion (2) and said dilator (3) are prevented when said tube (7) is inserted into said first sheath (5).

# F I G. 1

# F I G. 2

# F I G. 3

# F I G. 4

# F I G. 5

# F I G. 6

# F I G. 7